# EUROPÄISCHE PATENTSCHRIFT

(11) **EP 1 419 386 B1**
(45) Veröffentlichungstag und Bekanntmachung des Hinweises auf die Patenterteilung: **12.07.2006**
(21) Anmeldenummer: 02764836.9
(22) Anmeldetag: 07.08.2002
(51) Int. Cl.: G01N 33/48, A61K 9/16, A61K 9/14, G01N 33/543

(54) **VERFAHREN ZUR HERSTELLUNG VON PROTEIN-BELADENEN MIKROPARTIKELN**
METHOD FOR PRODUCING MICROPARTICLES LOADED WITH PROTEINS
PROCEDE DE PRODUCTION DE MICROPARTICULES CHARGEES DE PROTEINES

(30) Priorität: 10.08.2001 EP 01118812
(43) Veröffentlichungstag der Anmeldung: 19.05.2004
(73) Patentinhaber: Roche Diagnostics GmbH, 68305 Mannheim (DE); F.HOFFMANN-LA ROCHE AG, 4070 Basel (CH)
(72) Erfinder: FINKE, Andreas, 82377 Penzberg (DE); KLAUSE, Ursula, 82380 Peissenberg (DE); DONIE, Frederic, 82377 Penzberg (DE); HERRMANN, Rupert, 82362 Weilheim (DE); VON DER ELTZ, Herbert, 82362 Weilheim (DE); SLUKA, Peter, 82362 Weilheim (DE); JONA, Wolfgang, 84478 Waldkraiburg (DE)
(86) Internationale Anmeldenummer: PCT/EP2002/008789
(87) Internationale Veröffentlichungsnummer: WO 2003/019182

(56) Entgegenhaltungen:
- DE-A- 19 924 643
- SCHMIDT, H. H.-J. ET AL: "Quantification of lipoprotein A: comparison of an automated latex-enhanced nephelometric assay with an immunoenzymometric method." EUR. J. CLIN. CHEM. CLIN. BIOCHEM. (1996) 34(2), 119-124 CODEN: EJCBEO ISSN: 0939-4974, XP001029361
- CANTARERO L A ET AL: "THE ADSORPTIVE CHARACTERISTICS OF PROTEINS FOR POLYSTYRENE AND THEIR SIGNIFICANCE IN SOLID-PHASE IMMUNOASSAYS" ANALYTICAL BIOCHEMISTRY, ORLANDO, FL, US, Bd. 105, 1980, Seiten 375-382, XP001029367 ISSN: 0003-2697
- ILLUM L ET AL: "ATTACHMENT OF MONOCLONAL ANTIBODIES TO MICROSPHERES" METHODS IN ENZYMOLOGY, ACADEMIC PRESS INC, SAN DIEGO, CA, US, Bd. 112, 1985, Seiten 67-84, XP001029707 ISSN: 0076-6879

## Beschreibung

Die vorliegende Erfindung betrifft ein Verfahren zur Herstellung von Protein beladenen Mikropartikeln, welches dadurch charakterisiert ist, dass die Beladung der Mikropartikel in Suspension unter stark alkalischen Bedingungen erfolgt. Weiterhin betrifft die Erfindung die mit diesem Verfahren herstellbaren Mikropartikel und deren Verwendung in einem Bindungstest, z. B. in einem Immunoassay.

Protein beladene Mikropartikel sind bekannt und werden vielfach in medizinischen, immunologischen und diagnostischen Nachweisverfahren als Festphase verwendet. Die unbeladenen Ausgangspartikel (auch "Beads" genannt") bestehen vorwiegend aus Latex, beispielsweise Polystyrol-Latex, und sind oft durch einen Anteil an oder einem Kern aus Magnetit magnetisierbar. Die Kopplung von Protein an die Latexpartikel erfolgt bekanntermaßen in der Regel über chemische Linker (kovalente Bindung) oder durch Adsorption (nicht kovalente Bindung).

Die im Stand der Technik beschriebenen kovalenten Kopplungsverfahren gehen von Mikropartikeln (funktionalisierten Partikel) aus, die verschiedene funktionelle Gruppen (-COOH, -Tosyl, -Epoxy etc.) aufweisen. Sie nutzen diese funktionellen Gruppen zur Ausbildung der kovalenten Bindungen mit den Proteinen, z. B. über Amino- oder Carboxy-Gruppen der zu beschichtenden Proteine.

Kovalente Kopplungsverfahren unterscheiden sich von adsorptiven Kopplungsverfahren dadurch, dass die bei ersteren verwendeten funktionalisierten Partikel deutlich hydrophilere Oberflächen besitzen als nicht funktionalisierte Partikel. Dadurch wird der Anteil an adsorptiv gebundenem Protein reduziert. Adsorptiv gebundene Proteine können je nach Bindungsstärke eine Blutung verursachen. "Blutung" bedeutet, dass sich nicht oder schwach gebundenes Protein wieder ablöst. Dauerhaft wird nur Protein gebunden, welches mit den funktionellen Gruppen auf der Partikeloberfläche kovalent reagiert hat.

Allerdings weisen die Ausgangspartikel für kovalente Beschichtungsverfahren eine hohe Chargenvarianz bezüglich der Dichte an funktionellen Gruppen und geringe Lagerstabilität der funktionellen Gruppen auf der Oberfläche auf, was nach der Beladung zu niedrigen Beladungsdichten und/oder stark schwankenden Ergebnissen führt. Ein weiterer Nachteil kovalenter Beschichtungsverfahren ist die oft schlechte räumliche Zugänglichkeit der funktionellen Gruppen. Aus diesem Grund werden die für eine Verwendung, z. B. in einem sensitivem Immunoassay, mit den solchermaßen beschichteten Partikel notwendigen Beladungsdichten nicht erreicht. Die kovalente chemische Koppelung kann auch zu einer Inaktivierung des zu beschichtenden Proteins führen.

Giese offenbart in US 4,478,914 und US 4,656,252 Kopplungsverfahren, durch die eine mehrschichtige Beladung (Multilayer) von Oberflächen mit funktionellem Protein erreicht wird. Hierbei wurde Biotin kovalent an die Oberfläche gebunden und anschließend wiederholt Avidin und ein Biotin gekoppelter Extender an das zu beladene Material gebunden, wobei ungebundene Substanz jeweils durch Waschen entfernt wurde. Bei einem solchen Mehrschicht-Verfahren kann es durch verzögerte Desorption zu einer verspäteten Blutung kommen.

Für adsorptive Beschichtungsverfahren werden in der Regel Partikel mit einer hydrophoben Oberfläche eingesetzt. Bekannte Beispiele für solche Partikel sind Polystyrol-Partikel, die frei sind von den oben diskutierten funktionellen Gruppen oder Polystyrol-Partikel, die mit Polyurethan derivatisiert sind.

Die Erfahrung zeigt, dass Partikel, die über adsorptive Beschichtung hergestellt wurden, stärker zur Blutung neigen, als kovalent beschichtete Partikel. Ursächlich für dieses verstärkte Bluten ist die Tatsache, dass die adsorptive Beschichtung über relative schwache ionische und van der Waals Wechselwirkungen erfolgt.

Aus dem Stand der Technik sind eine Reihe von Kunstgriffen bekannt, um die adsorptive Bindung von Proteinen an Oberflächen zu verbessern und die Blutungstendenz zu reduzieren. DE 19924643 beschreibt die Beschichtung von Partikeln bei erhöhter Temperatur und der nachträglichen Bestrahlung mittels UV-Licht. Durch diese Maßnahmen wird die Blutungstendenz reduziert.

Conradie, J. D., et al., J Immunol Methods 59 (1983) 289-99 berichten über effizientere Beschichtung von Mikrotiterplatten bei Verwendung erhöhter Temperatur, erhöhter Salzkonzentrationen oder unter sauren pH-Bedingungen.

Ishikawa, E., et al., J Immunoassay 1 (1980) 385-98 beschreiben ebenfalls die vorteilhafte Verwendung von stark sauren pH-Bedingungen. Dort wird zur Beschichtung von Polystyrol-Kügelchen die Vorbehandlung von Antikörper bei pH 2,5 empfohlen.

Ein zusätzlicher Nachteil zu den aus dem Stand der Technik bekannten Verfahren ist in der starken Aggregationsneigung der eingesetzten hydrophoben Partikel zu sehen. Die hydrophobe Grundstruktur der Partikel führt dazu, dass diese Partikel einerseits untereinander verstärkt zur Ausbildung von Aggregaten neigen, welche sich nachteilhaft auf die Messgenauigkeit in den nachgeordneten Bestimmungsverfahren unter Verwendung dieser Partikel auswirkt. Andererseits zeigen diese Partikel ebenfalls aufgrund ihrer hydrophoben Natur beim Einsatz in immunologischen Test eine verstärkte Neigung zur unspezifischen Bindung von Probenbestandteilen. Unspezifische Bindungen in solchen Testsystemen wirken sich bekanntermaßen negativ auf Testcharakteristika, wie z. B. das Signal/Rauschen-Verhältnis aus, oder können zu falsch-positiven bzw. auch zu falschnegativen Ergebnissen führen.

Bei herkömmlichen Waschschritten werden die beladenen Teilchen durch zentrifugieren oder magnetische Abtrennung pelletiert und anschließend wieder resuspendiert. Die Zentrifugation oder Pelletierung bewirkt, dass sich die beladenen Partikel stark annähern und, im ungünstigen Fall, Aggregate bilden.

Aufgabe der vorliegenden Erfindung war es demnach, ein Verfahren bereit zu stellen, mit dem die großtechnische Herstellung von adsorptiv beladenen Mikropartikeln ermöglicht wird. Weiterhin beabsichtigt die Erfindung, proteinbelandenen Mikropartikel bereit zu stellen, die über Beladung unter stark alkalischen Bedingungen erhältlich sind und eine hohe Bindekapazität bei gleichzeitig geringer Blutung und guter Lagerungsstabilität aufweisen. Die Erfindung erstreckt sich ebenfalls auf die vorteilhafte Anwendung der unter alkalischen Bedingungen beschichteten Mikropartikeln in immunologischen Nachweissystemen.

Gemäß einer Ausführungsform der Erfindung wird diese Aufgabe durch ein Verfahren zur Herstellung von proteinbeladenen Mikropartikeln gelöst, das dadurch gekennzeichnet ist, dass die Beladung unter stark alkalischer pH-Bedingung erfolgt. Bevorzugterweise erfolgt die Beschichtung bei einem pH-Wert, der zwischen pH 10,0 und pH 12,5 gewählt wird.

Die zu beladenden Mikropartikel (oder Beads), die für das Verfahren der vorliegenden Erfindung verwendet werden können, sind mikrodispers und werden in Suspensionen mit Konzentrationen von unter 20 %, bevorzugt unter 10 % Gewicht pro Volumen verwendet, wobei ein Bereich von 0,1 bis 10 % Gew./Vol. insbesondere von 0,2 bis 2 % Gew./Vol. bevorzugt ist.

Im Gegensatz zum Stand der Technik, lassen sich gemäß vorliegender Erfindung sowohl hydrophobe, wie auch mit Epoxigruppen funktionalisierte Partikel vorteilhaft in einem Verfahren zur adsorptiven Beschichtung einsetzen.

Die Partikel können aus Latex und ähnlichen Materialien bestehen, bevorzugt aus Polystyrol-Latex und gegebenenfalls magnetisierbares Material wie etwa Magnetit enthalten. Die Partikelgröße der unbeladenen Teilchen liegt bevorzugt im Bereich von 50 nm bis 25 µm. Im Falle von Magnetpartikeln liegt die bevorzugte Größe im Bereich von 0,5 µm bis 25 µm, da in diesem Bereich die Magnetseparation günstig funktioniert. Geeignet sind beispielsweise Dynabeads der Firma Dynal mit einer Größe von ca. 2,8 µm, bestehend aus 88 % Polystyrol und 12 % Magnetit, wie z. B. die hydrophoben Beads M-280 oder die Epoxy-Beads M-270.

In einer bevorzugten Ausgestaltung ist das erfindungsgemäße Beschichtungsverfahren weiter dadurch gekennzeichnet, dass die verwendeten Polystyrol-Partikel eine hydrophobe oder eine mit Expoxidgruppen funktionalisierte Oberfläche aufweisen.

Die Verwendung von Polystyrol-Partikeln, die einen magnetisierbaren Kern aufweisen, ist besonders bevorzugt.

Das auf die Partikel zu bindende, bevorzugt polymerisierte Proteinmaterial hat eine Größe von mindestens 10 nm bis maximal 300 nm, bestimmt durch Photonenkorrelationsspektroskopie (PCS) (Lagaly, G., et al. Photonenkorrelationsspektroskopie in "Dispersionen und Emulsionen" (1997) 289 - 294, Darmstadt, Steinkopf), wobei ein Größenbereich von 20 nm bis 250 nm bevorzugt ist. Prinzipiell ist es möglich, Proteine mit einem Molekulargewicht von mehr als 10 kD zur Beladung der Mikropartikel einzusetzen. Bevorzugterweise handelt es sich bei dem zu beschichtenden Protein um einen Partner eines bioaffinen Bindepaares. Bioaffine Bindepaare sind z. B. Antigen/Antikörper, Hapten/Antikörper und Ligand/Rezeptor.

Ein Partner eines solchen Bindepaares, bevorzugterweise ein Antikörper oder ein Rezeptor wird zur Beschichtung eingesetzt. Ein Rezeptor für Biotin, wie das Avidin und insbesondere das Streptavidin, ist besonders bevorzugt. Für die vorliegende Erfindung besonders geeignete Materialien sind hochmolekulare Proteine oder polymerisierte Proteine, wie etwa polymerisiertes Streptavidin (SA-Poly). Es wurde gefunden, dass polymerisierte Proteine stärker adsorptiv auf Oberflächen binden. Der Grund ist wahrscheinlich, dass polymerisierte Proteine eine größere Anzahl an Kontaktstellen aufweisen. Somit wären für diese Polymere immer noch eine ausreichende Bindung an die Oberfläche gewährleistet, auch wenn sich einzelne Kontaktstellen lösen. Bei Monomeren mit wenigen bis einer einzigen Kontaktstelle löst sich das ganze Monomer ab, sobald sich die Kontaktstelle löst.

Polymerisierung kann in bekannter Weise bei Streptavidin durch chemische Behandlung erreicht werden. Bevorzugterweise wird in einem erfindungsgemäßen Beschichtungsverfahren polymerisiertes Avidin oder Streptavidin, besonders bevorzugt polymerisiertes Streptavidin, eingesetzt. Ebenfalls besonders geeignet sind polymerisierte Antikörper.

Wie eingangs erwähnt, stellt das Abbluten ein großes Problem vor allem während der Langzeitlagerung und während des Transports von Partikeln dar. Die Blutung wird bestimmt, indem die Partikel aus der eingelagerten Suspension abgetrennt und der Gehalt an abgeblutetem Bindepartner im Überstand gemessen wird.

Für Partikel, die mit Streptavidin beschichtet wurden, wird die Blutungsneigung in Nanogramm Streptavidin pro Milligramm Mikropartikel [ng/mg] angegeben. D. h., die Bindefähigkeit des abgebluteten Streptavidins wird mittels einer Eichkurve, die über monomeres Streptavidin ermittelt wurde, bestimmt. Bevorzugterweise liegt die Blutung der erfindungsgemäß hergestellte SA-Beads bei < 150 ng/mg und besonders bevorzugt bei < 80 ng/mg.

Wie schon erwähnt, ist es ein zentrales Element der vorliegenden Erfindung, dass die Beschichtung unter stark alkalischen pH-Bedingungen erfolgt. Als besonders geeignet hat sich der pH-Bereich von pH 10,0 bis pH 12,5 erwiesen. Je nach Zeitdauer des Beschichtungsverfahrens können sich unterschiedliche Unterbereiche aus diesem pH-Spektrum als optimal erweisen. Im grosstechnischen Maßstab wird die Beschichtung bevorzugterweise über einen Zeitraum von 1 - 10 Tagen vorgenommen. Besonders bevorzugt ist es, die Beschichtung über 4 - 7 Tage vorzunehmen. Bei Verwendung dieser relativ langen Zeitintervalle sind pH-Bereiche von 10,5 bis 12,5 und insbesondere von 11,0 bis 12,0 ganz besonders bevorzugt. Bei einer Verwendung von kürzerer Beschichtungszeiten können sich erhöhte pH-Optima ergeben.

Es hat sich ebenfalls als vorteilhaft erwiesen, die Salzkonzentration des zur Beschichtung alkalischen Puffers im physiologischen Salzbereich oder darüber anzusiedeln. Bevorzugt erfolgt die Beschichtung mit einem Puffer, der einen Salzgehalt von 0,1 bis 2 M, besonders bevorzugt von 0,3 bis 1,5 M und insbesondere bevorzugt von 0,8 bis 1,2 M.

Das In-Kontakt-bringen der Mikropartikelsuspension mit dem hochmolekularen Proteinmaterial in einem Verfahren gemäß der vorliegenden Erfindung erfolgt bevorzugt bei 15 - 30° C, besonders bevorzugt bei Raumtemperatur, also bei ca. 18 - 25° C, d. h. das Proteinmaterial wird nicht vortemperiert. Nach der Beladung unter stark alkalischen Pufferbedingungen können bereits ein erster oder mehrere Abtrennschritt(e) erfolgen, die dazu dienen, schwach oder nicht adsorbiertes Protein zu entfernen.

Die Abtrennung kann durch herkömmliche Verfahren, wie beispielsweise magnetische Trennung im Falle von Magnetit enthaltenden Mikropartikeln, durchgeführt werden. Bevorzugt ist für das Verfahren der vorliegenden Erfindung eine Abtrennung in einer Mikrofiltrationseinheit durch Siebe, Filter oder Membranen. Diese können sowohl hydrophil als auch hydrophob sein, es ist jedoch im letzteren Fall bevorzugt, sie vor dem Einsatz in einen hydrophilen Zustand zu überführen, was auf herkömmlich Art und Weise geschehen kann. Sie weisen bevorzugt eine Porengröße auf, welche zwischen der Größe der Mikropartikel und der Größe des hochmolekularen Proteinmaterials liegt. Besonders geeignete Porengrößen liegen im Bereich von etwa 50 % über der Größe des abzutrennenden Proteins, d.h. ca. 50 nm bis 2,5 µm. Membranen mit Porengrößen von 0,4 µm, bevorzugt 0,45 µm bis 2,5 µm, bevorzugt bis 2 µm sind besonders geeignet.

Die Abtrennung kann einmal oder mehrmals durchgeführt werden, wobei ein Puffer oder ein Puffersystem, bestehend aus unterschiedlichen Puffern, verwendet werden kann. Die Puffer enthalten bevorzugt Salze und waschaktive Substanzen zur Verdrängung/Solubilisierung nicht oder nur schwach gebundener Proteine, sowie sogenannte "blocking agents" (z.B. Serumalbumin) zum Auffüllen noch freier Bereiche der Partikeloberfläche. Bevorzugt wird der Abtrennschritt mehrmals, vorzugsweise dreimal, mit unterschiedlichen Puffern durchgeführt, die sich jeweils im Salz- und Detergenzgehalt voneinander unterscheiden. Die Abtrennung erfolgt unter einem Volumenaustausch von dem 5- bis 15-fachen des Ansatzvolumens. Wichtig für die Effektivität der Abtrennung ist sowohl die Abtrennzeit (Zeitspanne, in der die Partikel in einer bestimmten Pufferlösung suspendiert sind) als auch der Fluss und Druck und deren Kombination im Abtrennsystem. Fluß und Druck sind abhängig von der jeweils verwendeten Anlage und können vom Fachmann ermittelt werden. Abgetrennter Puffer kann durch Messung der Füllstandshöhe festgestellt und entsprechend durch frischen Puffer ersetzt werden.

Es wird bevorzugt während des gesamten Verfahrens darauf geachtet, dass eine Sedimentation der Mikropartikel verhindert wird. Besonders vorteilhaft ist es, zudem eine Sedimentation während der Abtrennung zu vermeiden. Zu diesem Zweck muss die Suspension in geeigneter Weise bewegt werden, was beispielsweise durch rühren, umpumpen, einleiten von Dispergierenergie oder jedweder Kombination solcher Maßnahmen oder durch andere geeignete physikalische Methoden erreicht werden kann.

Die Abfüllung der beladenen Partikel erfolgt ebenfalls bevorzugt auf sterile Art und Weise in einem dafür vorgesehenen Abfüllmodul.

Ein weiterer Gegenstand der Erfindung sind beschichtete Mikropartikel, die durch das zuvor beschriebene Verfahren erhältlich sind.

Gegenstand der Erfindung ist auch ein Verfahren zum Nachweis eines Analyten in einer Probe durch Zusammenbringen der Probe mit einem oder mehreren analytspezifischen Bindepartnern, wobei das Verfahren dadurch gekennzeichnet ist, dass ein erfindungsgemäß, mit einem Partner eines bioaffinen Bindepaares beschichteter Mikropartikel eingesetzt wird. Das Verfahren wird bevorzugt als Immunoassay durchgeführt. Das heißt, dass mindestens einer der analytspezifischen Bindepartner ein immunologischer Bindepartner ist. In diesem Verfahren wird die Probe, in der der Analyt vermutlich enthalten ist, mit einem immunologisch spezifischen Bindepartner inkubiert. Dieser immunologisch spezifische Bindepartner ist im Falle eines Antigen-Nachweises wie beispielsweise bei Tumormarkern wie PSA ein Antikörper oder ein Fragment davon, das spezifisch an den Analyten, das heißt das Tumorantigen PSA bindet. Bei Verfahren zum Nachweis von Antikörpern gegen ein bestimmtes Antigen (z.B. Anti-HCV-Antikörper) kann beispielsweise als immunologisch spezifischer Bindepartner das entsprechende Antigen eingesetzt werden.

Bevorzugterweise ist ein immunologischer Bindepartner mit dem zweiten Partner des zur Beschichtung eingesetzten bioaffinen Proteins konjugiert. Falls Streptavidin zur Beschichtung verwendet wurde, so ist der immunologische Bindungspartner biotinyliert. Nach Durchführung der üblichen und dem Fachmann geläufigen Inkubationsschritte, können die Partikel aus der Probe abgetrennt und die Menge an gefundenem Analyt in bekannter Weise bestimmt werden.

Ein weiterer Gegenstand der Erfindung ist daher die Verwendung erfindungsgemäß beschichteter Mikropartikel in einem Immunoassay.

In der Routinediagnostik sind die Bestrebungen zur Vereinfachung von Testführung (möglichst wenig Inkubationsschritte mit möglichst wenig Fehlermöglichkeiten) und Komplettversorgung (alle wesentlichen Testkomponenten aus einer Hand, möglichst in einer Packung) sehr weit fortgeschritten. Üblicherweise wird dem Kunden ein Satz an Reagenzien (ein Kit) geliefert, der alle testrelevanten Bestandteile enthält. Ein weiterer Gegenstand der Erfindung ist deshalb ein Test-Kit, das eine Suspension aus Mikropartikeln enthält, die gemäß vorstehender Erfindung beschichtet werden.

Die folgenden Beispiele, Publikationen und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung der Figuren

### Figur 1: pH-Abhängigkeit der Beschichtung

Dargestellt ist die pH-Abhängigkeit der Beschichtung für Dynal M-270 Beads. Bei Verwendung der unter verschiedenen pH-Bedingungen beladenen Beads unter ansonsten gleichen Testbedingungen in einem TSH-Test zeigt sich ein starker Anstieg des Meßsignals im pH-Bereich von pH 9.0 bis pH 12.5.

### Figur 2: Partikelverteilung bei Beschichtung von M-280-Beads gemäß Standardverfahren

Dynal M-280 Beads wurden, wie in DE 19924643 beschrieben, beschichtet. Mit dem Partikelzählgerät (Coulter Multisizer II) wurde die Partikelverteilung untersucht. Der Hauptpeak bei ca. 2.8 µm entspricht den monodispersen Partikeln, während die Nebenpeaks dimere Aggregate und Aggregate aus drei und mehr Partikeln darstellen.

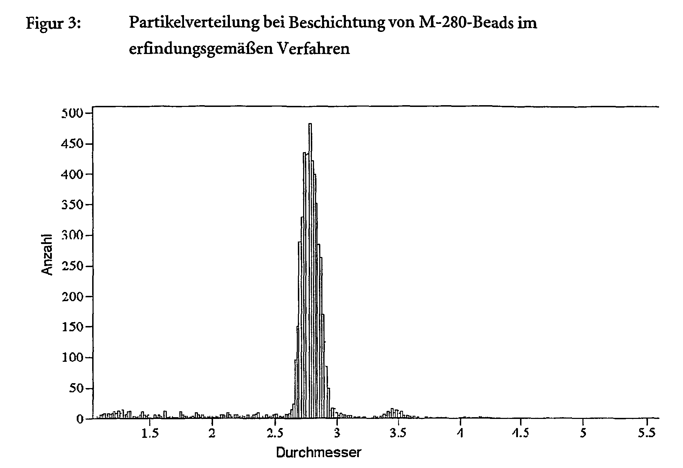

### Figur 3: Partikelverteilung bei Beschichtung von M-280-Beads im erfindungsgemäßen Verfahren

Dynal M-280 Beads wurden, wie in Beispiel 1 beschrieben, beschichtet. Mit dem Partikelzählgerät (Coulter Multisizer II) wurde die Partikelverteilung untersucht. Die Analyse zeigt eine sehr geringe Menge dimerer Aggregate. Größere Aggregate sind nicht evident.

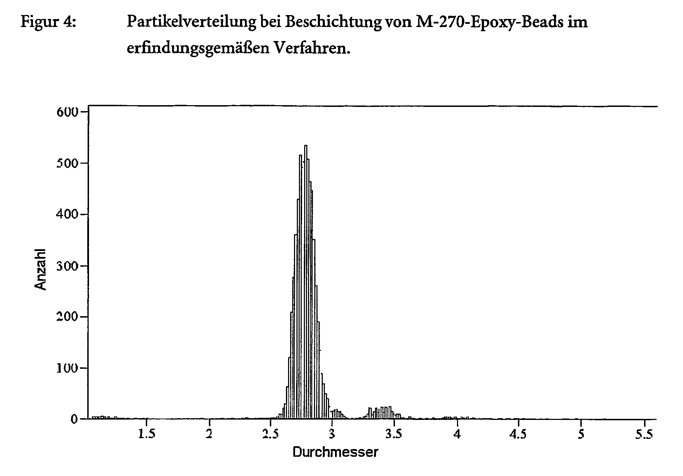

### Figur 4: Partikelverteilung bei Beschichtung von M-270-Epoxy-Beads im erfindungsgemäßen Verfahren.

Dynal M-280 Beads wurden, wie in Beispiel 1 beschrieben, beschichtet. Mit dem Partikelzählgerät (Coulter Multisizer II) wurde die Partikelverteilung untersucht. Die Analyse zeigt eine sehr geringe Menge dimerer Aggregate. Größere Aggregate sind nicht evident.

### Beispiel 1: Bead-Beschichtung

### 1.1 Referenz-Beads

Dynal M-280 Beads wurden, wie in DE 19 92 46 43 beschrieben, beschichtet.

### 1.2 Alkalische Beschichtung (pH-Optimierung)

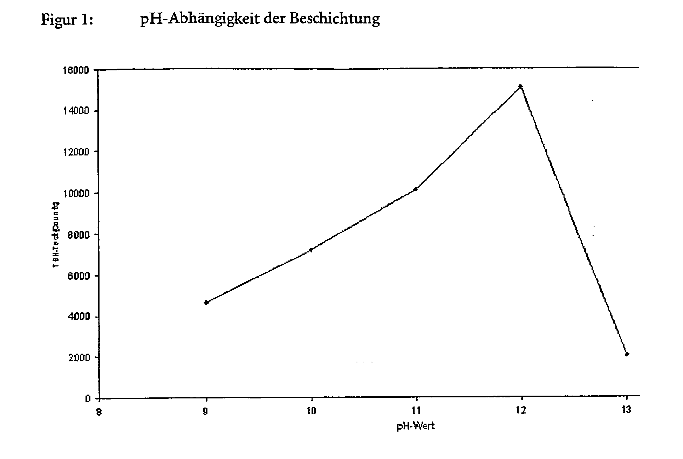

50 mg Magnetbeads (Dynal M-270 oder Dynal M-280) wurden nacheinander zunächst mit Isopropanol, danach mehrfach mit jeweils 5 ml 50 mM Na-Phosphat-Puffer (pH 9,0) gewaschen. Die Beads wurden anschließend in 4 ml 50 mM Na-Phosphat-Puffer (pH 9,0) resuspendiert. Anschließend wurde 10 mg polymerisiertes Streptavidin (poly-SA), gelöst in 1 ml 50 mM Na-Phosphat-Puffer pH 6.3 zugegeben. Der pH-Wert wurde anschließend mit NaOH auf den gewünschten Wert zwischen pH 10,0 und pH 12,5 eingestellt. Der Ansatz wurde 72 Stunden auf dem Rollenmischer inkubiert. Anschließend wurden die Beads abwechselnd mit 40 mM Na-Phosphat-Puffer (pH 7,4) und 1-prozentiger Tween20®⁻Lösung (im gleichen Grundpuffer) gewaschen. Die Beads wurden anschließend mehrmals mit einer Lösung bestehend aus Natrium-Phosphat-Puffer (10 mM Na-Phosphat 0,15 M Nall, pH 7,4) und RSA [0,5% w/v] inkubiert und gewaschen. Im Anschluß daran wurden Beads mit 50 mM HEPES-Puffer (pH 7,4) gewaschen und auf eine Endkonzentration von 0,7 mg/ml in diesem HEPES-Puffer (pH 7,4) eingestellt. Die Eignung der beschichteten Partikel für den Einsatz in einem Immuno-Assay wurde im TSH-Test (thyroid stimulating hormone = TSH) bestimmt und ist in Abbildung 1 für die M-270-Partikel graphisch dargestellt. Es ist offenkundig, daß die gewählten alkalischen Bedingungen zu einer deutlichen Verbesserung der Counts und damit der Eignung der Partikel im Test führen.

### 1.3 Alkalische Beschichtung (Routine-Methode)

50 mg Magnetbeads (Dynal M-270 oder Dynal M-280) werden nacheinander zunächst mit Isopropanol, danach mehrfach mit jeweils 5 ml 50 mM Na-Phosphat-Puffer (pH 9,0) gewaschen. Die Beads werden anschließend in 4 ml 50 mM Na-Phosphat-Puffer (pH 9,0) resuspendiert. Anschließend wird 10 mg polymerisiertes Streptavidin (poly-SA), gelöst in 1 ml 50 mM Na-Phosphat-Puffer pH 6,3 zugegeben. Der pH-Wert wird anschließend mit NaOH auf den gewünschten Wert zwischen pH 10,0 und pH 12,5 eingestellt. Der Ansatz wird 4 bis 7 Tage auf dem Rollenmischer inkubiert. Anschließend werden die Beads abwechselnd mit 40 mM Na-Phosphat-Puffer (pH 7,4) und 1-prozentiger Tween20®⁻Lösung gewaschen. Die Beads werden anschließend mehrmals mit einer Lösung bestehend aus Natrium-Phosphat-Puffer (10 mM Na-Phosphat, 0,15 M Nall, pH 7,4) und RSA [0,5% w/v] inkubiert und gewaschen. Im Anschluß daran werden Beads mit 50 mM HEPES-Puffer (pH 7,4) gewaschen und auf eine Endkonzentration von 0,7 mg/ml in diesem HEPES-Puffer eingestellt.

### Beispiel 2: Partikelverteilung

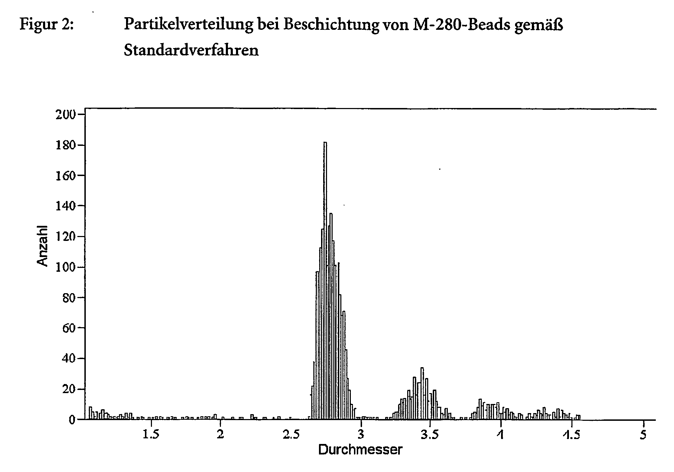

Mit einem Partikelzählgerät (Coulter Multisizer II) wurden die mit dem oben beschriebenen Verfahren beschichteten Partikel Dynal M-280 und M-270 Beads mit nach herkömmlichen Verfahren beschichteten M-280 Beads verglichen (Figuren 2 - 4). Bei alkalischer Beschichtung resultiert eine deutlich homogenere Verteilung der beschichteten Beads mit wesentlich geringeren Anteilen an dimeren, trimeren und höheren Aggregaten. Dies geht aus dem Vergleich von Abbildungen 3 und 4 zu Abbildung 2 hervor.

### Beispiel 3: Blutungsverhalten

Das Abbluten beschichteter Proteine erfolgt unter normalen Lagerbedingungen (4 - 8° C) über viele Monate hinweg sehr langsam. Um die Abblutung unter Lagerbedingungen zu simulieren, wird ein Kurzzeitmodel eingesetzt. Die beschichteten Partikel werden hierzu bei 35° C über 21 Tage auf dem Rollenmischer gelagert. Die Biotinbindekapazität des Überstandes, die durch abgeblutetes Streptavidin bedingt ist, wird z. B. unter Verwendung von radioaktiv markiertem Biotin bestimmt.

Die Blutungsneigung wird in ng SA/mg Partikel angegeben. Zur Ermittlung der Blutung wird eine Standardkurve mittels monomerem Streptavidin erstellt und die Biotinbindekapazität des Überstandes belasteter Mikropartikelsuspensionen daran abgelesen. Für die gemäß im Beispiel 1 beschichteten Mikropartikel wurden überraschend geringe Blutungsneigungen festgestellt, die regelmäßig unter 150 ng/mg, in den meisten Fällen sogar unter 100 ng/mg nach Belastung lagen.

### Beispiel 4: Ergebnisse im HIV-Funktionstest

Es wurde ein Doppel-Antigen-Brückentest zum Nachweis spezifischer Antikörper gegen HIV durchgeführt. Hierbei wurde die Probenflüssigkeit mit einem biotinylierten Antigen in Gegenwart einer Streptavidin-beschichteten Festphase (magnetische SA-Beads nach Standardverfahren bzw. gemäß dem hier beanspruchten Verfahren) inkubiert. Nach einem Waschritt wurde nochmals das gleiche Antigen, diesmal jedoch Ruthenium-markiert, zugegeben. Das Vorhandensein von Anti-HIV-Antikörpern in der Probenflüssigkeit wurde durch Bestimmung der Ruthenium-Markierung an der Festphase an Hand der Elektrochemilumineszenz im Elecsys® -System bestimmt.

Als Antigen wurde ein HIV-Peptid aus dem gp36-Bereich von HIV 2 (nähere Beschreibung hierzu ist aus WO 96/03652 zu entnehmen) verwendet, das am N-Terminus gelabelt ist. Die Konzentration an Antigen betrug im Test:
6 ng/ml biotinyliertes Ag, 200 ng/ml ruthenyliertes Ag.

Der Systemleerwert wurde in Abwesenheit von ruthenyliertem Antigen bestimmt.

In den folgenden Tabellen ist das Ergebnis der Experimente mit den erfindungsgemäß beschichteten SA-Beads basierend auf den Dynal M-280 Ausgangspartikeln im Vergleich zu Partikeln dargestellt, die gemäß Stand der Technik beschichtet wurden.

**Tabelle 1: HIV-Test/Meßwerte**

| [counts] | Streptavidin-Beads gemäß Beispiel 1 | Streptavidin-Beads gemäß Stand der Technik |
|---|---|---|
| Systemleerwert | 316 | 481 |
| Negative Probe | 560 | 2.150 |
| Positive Probe 1 | 2.442.861 | 2.506.269 |
| Positive Probe 2 | 1.073.515 | 1.338.978 |
| Positive Probe 3 | 7.315 | 11.802 |

Die Signaldynamik wurde nach folgender Formel berechnet:
(Signal ― Systemleerwert) / (Neg.-Probe - Systemleerwert)

**Tabelle 2: HIV-Test/Signaldynamik**

| Signaldynamik Pos./neg. Seren | Streptavidin-Beads gemäß Beispiel 1 | Streptavidin-Beads gemäß Stand der Technik |
|---|---|---|
| Negative Probe | 1,0 | 1,0 |
| Positive Probe 1 | 10.010,4 | 1.501,4 |
| Positive Probe 2 | 4.398,4 | 802,0 |
| Positive Probe 3 | 28,7 | 6,8 |

Es ist ersichtlich, dass durch die Verwendung der erfindungsgemäßen SA-Beads deutlich niedrigere Leerwerte und damit verbunden eine bessere Differenzierung von Positiv- zu Negativsignal ergibt.

Die nächste Tabelle zeigt die unspezifische Bindung des Antikörper-Konjugats an die Streptavidin-Festphase. Bei dieser Testführung wird anstelle der biotinylierten Antigene nur Puffer eingesetzt. Die Konzentration des Ruthenium-Konjugats beträgt 600 ng/ml.

**Tabelle 3: Unspezifische Bindung des Ru-Konjugates aus dem HIV-Test**

| [counts] | Streptavidin-Beads gemäß Beispiel 1 | Streptavidin-Beads gemäß Stand der Technik |
|---|---|---|
| Systemleerwert | 316 | 481 |
| Puffer | 446 | 2.061 |
| Humanserum | 373 | 692 |

Die erfindungsgemäßen Beads zeigen auch hier die besseren Leerwerte (geringere unspezifische Bindung) und eine deutlich reduzierte Matrixabhängigkeit (geringere Signalunterschiede zum Systemleerwert bei analytfreien Proben), was präzisere Tests zuläßt.

### Beispiel 5: Ergebnisse im CA 15-3 Test

Beim verwendeten CA 15-3 Test handelt es sich um einen Sandwich-Assay im 2-stufigen Testformat. Im ersten Schritt werden Beads, Probe und der biotinylierte Antikörper inkubiert, anschließend die Beads durch Anlagerung an Magneten separiert und der Überstand abgesaugt. Nach mehreren Waschschritten (Zugabe von Waschpuffer, erneutes Absaugen, Resuspendieren durch Vortexen in Waschpuffer, erneutes Separieren, Absaugen) werden die Beads in Reagenz 2, das den ruthenylierten Antikörper enthält, resuspendiert. Nach einer weiteren Inkubation wird erneut gewaschen, danach werden die in Waschpuffer resuspendierten Beads in die Meßzelle eines Elecsys® E1010-Gerätes überführt und die Menge des über den Analyten gebundenen Ruthenium-Labels bestimmt.

Im CA 15-3 Test stellt die Bead-Aggregation ein besonderes Problem dar; der Analyt ist ein Antigen mit vielfach repetitiven Elementen, die die Bindung mehrerer Antikörper-Bead-Komplexe ermöglichen. Während der Waschschritte werden die Beads auf relativ kleinen Flächen abgelagert und kommen somit in engen räumlichen Kontakt. Dies kann sogar die Ausbildung dreidimensionaler Komplexe zur Folge haben. Diese Verklumpungen zu größeren Aggregaten sind auf der Meßzelle sichtbar und führen zu schlechten Präzisionen (hoher Variationskoeffizient) und geringen Signalausbeuten.

Die Verklumpungen sind in Farbabbildungen gut sichtbar, kommen dagegen in Schwarz/Weiß Abbildungen in unterschiedlichen Graustufen kaum zur Geltung, weshalb auf eine entsprechende Figur verzichtet wurde. Die beschichteten Beads selbst haben einen großen Einfluß auf das Ausmaß der Verklumpungen auf der Meßelektrode. Im Gegensatz zu den Standard SA-Beads zeigen erfindungsgemäß beschichtete Beads eine zum Teil deutlich verbesserte Verteilung auf der Meßzelle durch wesentlich geringere Aggregation. Dabei ist es unerheblich, ob für die Beschichtung M270 Epoxy-Beads oder die hydrophoben M280 Beads eingesetzt wurden. In beiden Fällen ergibt sich eine deutlich homogenere Verteilung auf der Elektrode im Vergleich zur Referenz.

Eine verbesserte Bead-Verteilung führt auch zu einer verbesserten Signalwiederfindung. In Tabelle 4 sind die entsprechenden Ergebnisse eines repräsentativen CA 15-3 Tests zusammengefasst.

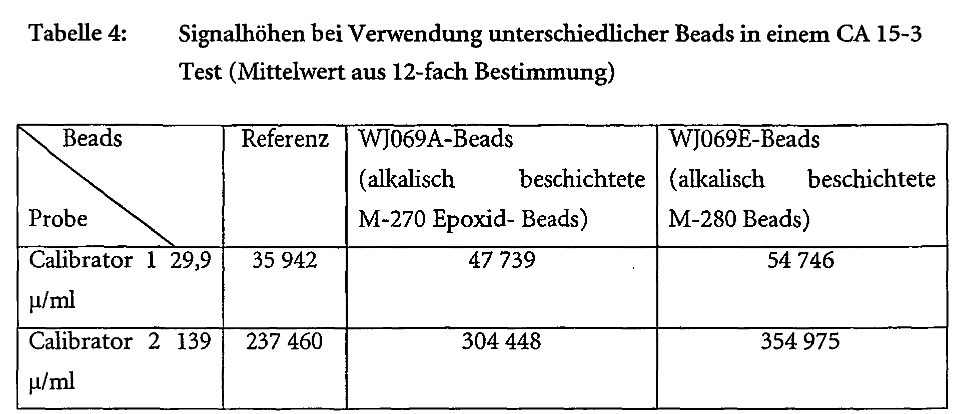

Aus dieser Tabelle wird ersichtlich, dass bei Verwendung identischer Einsatzstoffe der Einsatz unterschiedlicher Bead-Chargen im CA 15-3 Test zu unterschiedlichen Signalhöhen führt. Die Signalhöhe ist für die alkalisch beschichteten Partikel deutlich verbessert.

Die Bestimmung des Variationskoffizienten erfolgte mit dem CA 15-3 Test der Firma Roche Diagnostics GmbH (Bestell-Nr. 1776169) nach Angaben des Herstellers. An Stelle der beschichteten Beads aus der Testpackung wurden die in Tabelle 5 aufgeführten Bead-Präparationen eingesetzt.

Der Variationskoeffizient wird nach den üblichen statistischen Verfahren aus der 21-fachen Bestimmung der zugrunde liegenden Probe ermittelt.

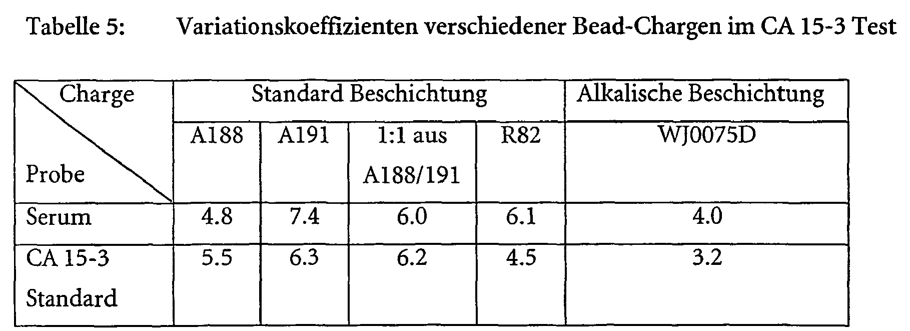

Die oben stehende Tabelle 5 zeigt exemplarisch, dass Partikel, die unter den erfindungsgemäßen alkalischen Beschichtungsbedingungen beladen wurden, im CA 15-3 Test einen deutlich geringeren Variationskoeffizienten (VK - ausgedrückt in % der gemessenen Counts) aufweisen. Je geringer der VK ist, desto präziser kann die Bestimmung des Analytmoleküls erfolgen.

### Referenzliste

DE 19924643
US 4,478,914
US 4,656,252
WO 96/03652
Lagaly, G., et al. Photonenkorrelationsspektroskopie in "Dispersionen und Emulsionen" (1997) 289 - 294, Darmstadt, Steinkopf
Conradie, J. D., et al., J Immunol Methods 59 (1983) 289-99
Ishikawa, E., et al., J Immunoassay 1 (1980) 385-98

## Patentansprüche

1. Verfahren zur Herstellung beschichteter Polystyrol-Partikel, **dadurch gekennzeichnet, dass** die adsorptive Beschichtung der Polystyrol-Partikel mit einem Partner eines bioaffinen Bindepaares bei einem pH-Wert im Bereich von pH 10,0 bis pH 12,5 erfolgt.

2. Verfahren nach Anspruch 1, **dadurch gekennzeichnet, dass** besagter Partner eines bioaffinen Bindepaares in polymerisierter Form vorliegt.

3. Verfahren nach Anspruch 1 oder 2, **dadurch gekennzeichnet, dass** besagter Bindepartner ein polymerisiertes Streptavidin ist.

4. Verfahren nach einem der Ansprüche 1 bis 3, **dadurch gekennzeichnet, dass** die verwendeten Polystyrol-Partikel eine hydrophobe oder eine mit Epoxidgruppen funktionalisierte Oberfläche aufweisen.

5. Verfahren nach einem der vorstehenden Ansprüche, **dadurch gekennzeichnet, dass** die verwendeten Polystyrol-Partikel einen magnetisierbaren Kern aufweisen.

6. Beschichtetes Polystyrol-Partikel erhältlich nach einem Verfahren gemäß einem der Ansprüche 1 bis 5.

7. Verwendung beschichteter Polystyrol-Partikel gemäß Anspruch 6 in einem Immunoassay.

8. Test-Kit, das eine Suspension aus beschichteten Polystyrol-Partikel gemäß Anspruch 6 enthält.

## Claims

1. Method for producing coated polystyrene particles, **characterized in that** the polystyrene particles are adsorptively coated with a partner of a bioaffine binding pair at a pH in the range of pH 10.0 to pH 12.5.

2. Method as claimed in claim 1, **characterized in that** the said partner of a bioaffine binding pair is present in a polymerized form.

3. Method as claimed in claim 1 or 2, **characterized in that** the said binding partner is a polymerized streptavidin.

4. Method as claimed in one of the claims 1 to 3, **characterized in that** the polystyrene particles that are used have a hydrophobic surface or a surface functionalized with epoxide groups.

5. Method as claimed in one of the previous claims, **characterized in that** the polystyrene particles have a magnetizable core.

6. Coated polystyrene particle obtainable by a method as claimed in one of the claims 1 to 5.

7. Use of coated polystyrene particles as claimed in claim 6 in an immunoassay.

8. Test kit containing a suspension of coated polystyrene particles as claimed in claim 6.

## Revendications

1. Procédé de préparation de particules de polystyrène revêtues, **caractérisé en ce que** le revêtement par adsorption des particules de polystyrène est réalisé avec un partenaire d'une paire de liaison présentant une bio-affinité dans la plage de pH 10,0 à pH 12,5.

2. Procédé selon la revendication 1, **caractérisé en ce que** ledit partenaire d'une paire de liaison présentant une bio-affinité se trouve sous forme polymérisée.

3. Procédé selon la revendication 1 ou 2, **caractérisé en ce que** ledit partenaire de liaison est une streptavidine polymérisée.

4. Procédé selon l'une quelconque des revendications 1 à 3, **caractérisé en ce que** les particules de polystyrène utilisées présentent une surface hydrophobe ou fonctionnalisée avec des groupes époxyde.

5. Procédé selon l'une quelconque des revendications précédentes, **caractérisé en ce que** les particules de polystyrène utilisées présentent un noyau magnétisable.

6. Particules de polystyrène revêtues pouvant être obtenues selon un procédé selon l'une quelconque des revendications 1 à 5.

7. Utilisation de particules de polystyrène revêtues selon la revendication 6 dans un immuno-essai.

8. Kit de test qui contient une suspension de particules de polystyrène revêtues selon la revendication 6.
